# EUROPÄISCHE PATENTSCHRIFT

(11) **EP 0 638 565 B1**
(45) Veröffentlichungstag und Bekanntmachung des Hinweises auf die Patenterteilung: **17.09.1997**
(21) Anmeldenummer: 94111828.3
(22) Anmeldetag: 29.07.1994
(51) Int. Cl.: C07D 307/33

(54) **Verfahren zur Herstellung von gamma-Butyrolacton**
Process for producing gamma-butyrolactone
Procédé de préparation de gamma-butyrolactone

(30) Priorität: 10.08.1993 DE 4326692
(43) Veröffentlichungstag der Anmeldung: 15.02.1995
(73) Patentinhaber: Akzo Nobel N.V., 6824 BM Arnhem (NL)
(72) Erfinder: Bergfeld, Manfred, Dr., D-63906 Erlenbach-Mechenhard (DE); Wiesgickl, Günther, Dr., D-91550 Dinkelsbühl (DE)
(74) Vertreter: Fett, Günter

(56) Entgegenhaltungen:
- EP-A- 0 184 055
- GB-A- 931 685
- US-A- 4 006 165

## Beschreibung

Die Erfindung betrifft ein Verfahren zur Herstellung von gamma-Butyrolacton durch katalytische Hydrierung von Maleinsäureanhydrid in der Dampfphase.

Gamma-Butyrolacton ist eine wichtige Chemikalie, die als Ausgangsstoff für zahlreiche Synthesen von Bedeutung ist, sie spielt zum Beispiel eine Rolle bei der Herstellung von Buttersäure und deren Derivate, Butandiol, Tetrahydrofuran, N-Methylpyrrolidon, Polyvinylpyrrolidon, Methionin und dergleichen. Gamma-Butyrolacton ist ferner ein wichtiges Lösungsmittel unter anderem für Acrylate und Polymere auf Styrolbasis. Es kann ferner als Lösungsmittel unter anderem bei der Herstellung von Synthese-Fasern eingesetzt werden.

Eine Reihe von Herstellungsmethoden gehen von Maleinsäureanhydrid bzw. Abkömmlingen wie Maleinsäure, Bernsteinsäureanhydrid, Maleinsäureester aus, die einer Hydrierung unterworfen werden. Die Hydrierung wird meistens in der Dampfphase und in Gegenwart von Katalysatoren durchgeführt. Es werden in der Patentliteratur zahlreiche Katalysatoren für diese Reaktionen beschrieben. So ist zum Beispiel der US-PS 3 065 243 ein Verfahren zu entnehmen, bei dem Kupferchromit als Katalysator dient. Wie der Beschreibung und den Beispielen dieser Patentschrift zu entnehmen ist, entstehen bei dieser Umsetzung noch beträchtliche Mengen an Bernsteinsäureanhydrid, das im Kreislauf gefahren werden muß.

Es hat nicht an Versuchen gefehlt, Katalysatoren zu entwickeln, um die Ausbeute und die Selektivität zu verbessern. Auch war es Ziel der Untersuchungen, die Lebensdauer der Katalysatoren zu verbessern, da bei vielen Katalysatoren die Standzeiten für den Dauerbetrieb zu kurz sind; es kommt bei Dauerbetrieb nämlich zu schnell zu einer Deaktivierung des Katalysators, meistens durch Verkokung des Katalysators.

So werden in der kanadischen Patentschrift Nr. 840 452 weiterentwickelte Katalysatoren beschrieben, die auf der Basis von Kupfer/Zink aufgebaut sind. Diese können zusammen mit Asbest zu entsprechenden Katalysatorteilchen verarbeitet werden. Sowohl der in dieser kanadischen Patentschrift beanspruchte Katalysator als auch der vergleichsweise hergestellte Kupferchromitasbest-Katalysator erfüllen noch nicht alle Wünsche, die an einen guten Katalysator für die Herstellung von gamma-Butyrolacton gestellt werden.

In der DE-OS 24 04 493 wird ein Verfahren beschrieben, bei dem in Gegenwart von Wasserdämpfen hydriert wird. Dadurch soll die Verkokung des Katalysators reduziert werden. Von Nachteil bei diesem Verfahren ist unter anderem, daß Wasser eine Verbindung ist, die nicht inert ist, d.h. die als Reaktionskomponente Teil des Gleichgewichts ist.

Weitere Katalysatoren auf Basis von Kupferchromit werden zum Beispiel in der US-PS 4 006 165 beschrieben, wobei dieser Katalysator noch Nickel enthalten muß. Diese Katalysatoren können auf Aluminiumoxyd oder Silica wie Kieselgur aufgebracht werden oder auch durch Mischung mit denselben hergestellt werden.

Obwohl bereits zahlreiche Katalysatoren für die Umsetzung von Wasserstoff und Maleinsäureanhydrid zu gamma-Butyrolacton beschrieben sind, besteht noch ein Bedürfnis nach Katalysatoren, mit denen sich diese Umsetzung auf bessere und vorteilhaftere Weise durchführen läßt.

Aufgabe der Erfindung ist es deshalb, ein Verfahren zur Herstellung von gamma-Butyrolacton durch Hydrieren von Maleinsäureanhydrid in der Dampfphase in Gegenwart eines Katalysators zur Verfügung zu stellen, das mit hohen Ausbeuten arbeitet, das eine hervorragende Selektivität besitzt, das sowohl unter erhöhtem Druck als auch bei normalem oder unteratmosphärischem Druck arbeiten kann, das wirtschaftlich arbeitet, das flexibel ist und das insbesondere Vorteile bei der Aufarbeitung des Reaktionsprodukts und bei der Rückführung bestimmter Komponenten in den Kreislauf bietet und das auch so gefahren werden kann, daß ein Rezyklieren von Bernsteinsäureanhydrid, das als Zwischenstufe entsteht, nicht erforderlich ist.

Diese Aufgabe wird gelöst durch ein Verfahren zur Herstellung von gamma-Butyrolacton durch katalytische Hydrierung von Maleinsäureanhydrid in der Dampfphase in Gegenwart von Katalysatoren auf der Basis von Kupferchromit in reduzierter Form, dadurch gekennzeichnet, daß man zur Durchführung der Reaktion einen im wesentlichen einheitlichen Katalysator verwendet, der sich im wesentlichen von den 3 Komponenten Kupferoxyd, Chromoxyd und Siliciumdioxyd ableitet. Vorzugsweise wird ein Katalysator auf der Basis von 68 - 85 Gew.% CuO, 15 - 30 Gew.% Cr₂O₃ und 0,5 - 5 % SiO₂ aufgebaut ist, verwendet, wobei diese Mengenangaben auf den noch nicht durch Reduktion vorbereiteten Katalysator bezogen sind. In einer besonder vorteilhaften Ausführungsform ist das Verhältnis CuO zu Cr₂O₃ zu SiO₂ etwa 78 : 20 : 2.

In einer besonders vorteilhaften Ausführungsform des erfindungsgemäßen Verfahrens wird die Hydrierung in Gegenwart eines inerten Gases als Verdünnungsmittel, vorzugsweise Stickstoff, durchgeführt. Außer Stickstoff können auch die bekannten Edelgase wie Argon, Krypton, Helium oder deren Gemische unter sich oder mit Stickstoff verwendet werden. Es versteht sich von selbst, daß der Katalysator auf Basis der 3 Komponenten in an sich bekannter Weise vor dem Einsatz in der Reaktion in üblicher Weise reduziert wird. Vorzugsweise wird die Reduktion im Reaktor selbst durchgeführt.

Die Reduktion kann zum Beispiel nach folgender Verfahrensweise durchgeführt werden. Der Katalysator, der als Katalysator-Bett vorliegt, wird im Reaktor im Stickstoffstrom auf 150 °C aufgeheizt. Bei dieser Temperatur wird langsam Wasserstoff bis zu einer Eingangskonzentration von bis zu 8 Vol.% eingespeist. Die Temperatur sollte dabei im Katalysator-Bett nicht stärker als um 25 °C steigen.

Nach Abklingen der Reaktionswärme wird die Wasserstoff-Konzentration auf 80 - 100 % durch Zurücknahme des Stickstoffstromesgesteigert und die Temperatur bis zu 280 °C angehoben. Die Temperatur wird 12 Stunden unter H₂-Durchfluß eingehalten; diesen Vorgang nennt man im allgemeinen Nachreaktion.

Selbstverständlich kann die Reduktion auf andere Weise geschehen, zum Beispiel wie in der US-PS 3 065 243 in Spalte 2, Zeilen 54 bis 66 angegeben.

Die Herstellung des einheitlichen Katalysators auf Basis der 3 Komponenten kann auf folgende Weise geschehen. Kupfer und Chrom in Form von gelösten Salzen werden mit einer entsprechenden löslichen Silicium-Verbindung, vorzugsweise in wäßrigem System miteinander vermengt. Als Silicium-Verbindung kann zum Beispiel Wasserglas oder Kieselsol verwendet werden. Auf geeignete Weise, zum Beispiel durch Alkalisieren fällt man Kupfer, Chrom und Silicium gemeinsam beispielsweise in Form der entsprechenden Oxide oder Hydroxide aus, so daß ein einheitlicher gleichmäßig aufgebauter Niederschlag entsteht. Nach Filtrieren und Waschen des Niederschlags wird der Niederschlag gegebenenfalls getrocknet und dann kalziniert. Die kalzinierten Massen werden entsprechend zerkleinert und gegebenenfalls auf entsprechende Korngrößen aufgeteilt, zum Beispiel durch Passieren durch mehrere Siebe.

Einheitlicher Katalysator im Rahmen der Erfindung bedeutet, daß die 3 Komponenten, aus denen der Katalysator sich ableitet, homogen mit einander verbunden sind, so wie es zum Beispiel der Fall ist, wenn man die Komponenten aus einer Lösung gemeinsam zur Fällung bringt, oder wenn man die 3 Komponenten gemeinsam aufschmilzt und erstarren läßt. Es gehören also nicht dazu grobkörnige Gemische der einzelnen Komponenten, bei der die einzelnen Komponenten noch als diskrete Teilchen vorliegen. Auch übliche Kupferchromitkatalysatoren auf Silica-Trägern zählen nicht zu den Katalysatoren gemäß der Erfindung. Selbstverständlich kann der erfindungsgemäße Katalysator auf Trägern aufgebracht werden.

Der Katalysator kann dann sogleich in den Reaktor eingebracht und nach entsprechender Reduktion für die Reaktion verwendet werden.

Die Hydrierung von Maleinsäueanhydrid, d.h. die Reaktion von Maleinsäureanhydrid mit Wasserstoff wird in der Dampfphase durchgeführt, d.h. bei erhöhten Temperaturen zum Beispiel in einem Bereich von etwa 100 - 400 °C, bevorzugt ist der Bereich von etwa 250 - 280 °C.

Das dampfförmige Maleinsäureanhydrid kann für sich selbst durch Erhitzen und Überführen in die Dampfphase und entsprechendes Dosieren in den Reaktionsraum geführt werden. Es ist aber auch möglich, die erforderlichen Mengen an Maleinsäureanhydrid-Dämpfen mittels des zu dosierenden Wasserstoffstromes in den Reaktor mitzuführen. Selbstverständlich kann dies auch durch das gegebenenfalls mitverwendete inerte Gas wie Stickstoff geschehen.

Das molare Verhältnis von Maleinsäureanhydrid zu Wasserstoff kann in dem Strom der Ausgangsprodukte in weiten Bereichen variieren und zum Beispiel zwischen 1 : 20 bis 1 : 250 liegen, der Bereich 1 : 50 bis 1 : 150 wird bevorzugt.

Die Reaktion kann sowohl bei normalem Druck als auch bei Unterdruck oder erhöhtem Druck durchgeführt werden wie zum Beispiel zwischen 0,1 bis 10 bar.

Inertes Gas bedeutet im Rahmen der Erfindung, daß es bei der Umsetzung nicht als Reaktionspartner oder Reaktionsprodukt teilnimmt und sich auch nicht selbst durch eine Reaktion verändert.

Durch Verwendung eines inerten Gases als Verdünnungsmittel ist es möglich, die Reaktion in günstiger Weise zu beeinflussen. Das Verhältnis von inertem Gas, zu dem vorzugsweise Stickstoff zu zählen ist, das aber auch eins der übrigen Edelgase wie Helium, Argon, Krypton und Xenon sein kann, wobei auch Gemische der Edelgase oder Gemische von Stickstoff und Edelgase verwendet werden können, kann ebenfalls in weiten Bereichen variieren. Es versteht sich von selbst, daß die Verdünnung, abhängig von den gewählten sonstigen Reaktionsbedingungen dort ihre Grenze findet, wo der Anteil des Verdünnungsmittels so groß ist, daß zu wenig Wasserstoff vorhanden ist und die Ausbeute bezogen auf Maleinsäueanhydrid stark abnimmt. Diese Grenze kann durch wenige einfache Versuche, die zum handwerklichen Können eines Durchschnittsfachmannes gehören, ermittelt werden.

Es ist möglich, die Reaktion durch Variieren der verschiedensten Verfahrensparameter zu beeinflussen. So kann die Verweilzeit geändert werden, was durch Einstellen verschiedener Dosiergeschwindigkeiten ermöglicht werden kann, was aber auch durch Verlängerung der Reaktionsstrecke bewerkstelligt werden kann, zum Beispiel durch Verwendung eines längeren Reaktionsrohres, das entsprechend mit Katalysator gefüllt ist.

Die Reaktion läßt sich so steuern, daß das als Zwischenstufe entstehende Bernsteinsäureanhydrid am Ende des Reaktors nicht mehr vorhanden ist und somit nicht mehr im Kreislauf gefahren werden muß. Andererseits läßt sich bei Bedarf die Reaktion auch so steuern, daß Bernsteinsäureanhydrid in mehr oder weniger großen Mengen noch in den austretenden Reaktionsprodukten vorhanden ist und dann entweder nach Abtrennung für sich selbst weiterverarbeitet wird oder auch wieder im Kreislauf gefahren wird.

Es war völlig überraschend, daß die erfindungsgemäße Verwendung des Katalysators sowohl eine hohe Selektivität als auch eine hohe Ausbeute bewirkt. Die Vorteile des erfindungsgemäß verwendeten Katalysators zeigen sich nicht nur beim Arbeiten mit oder ohne Verdünnungsmittel, sie zeigen sich auch, wenn man die molaren Verhältnisse der Reaktionspartner verändert, wenn man die Temperaturen variiert. Somit ist dieser Katalysator bei der Herstellung von gamma-Butyrolacton durch Reduktion von Maleinsäureanhydrid mittels Wasserstoff unter den verschiedensten Verfahrensbedingungen mit großen Vorteilen zu verwenden.

Zum Nachweis der völlig überraschenden, überlegenen Reaktionsweise des erfindungsgemäßen Katalysators werden neun verschiedene Katalysatoren auf der Basis von Kupferchromit hergestellt, wobei der Katalysator Nr. 4 der Erfindung entspricht, die übrigen Katalysatoren übliche bekannte Kupferchromit-Katalysatoren darstellen. Die Katalysatoren werden durch Fällen entsprechender Lösungen gewonnen. Die Zusammensetzung der eingesetzten Katalysatoren ist in Tabelle 1 gegeben, wobei die Zusammensetzung diejenige vor der Reduktion ist.

**Tabelle 1**

| Zusammensetzung (vor Reduktion) | Kat.-Nr. |
|---|---|
| 2 CuO * Cr₂O₃ | 1 |
| Cu-chromit (9.7 % BaO) | 2 |
| 80 % CuO, 20 % Cr₂O₃ | 3 |
| 78 % CuO, 20 % Cr₂O₃, 2 % SiO₂ | 4 |
| 42 % CuO, 38 % Cr₂O₃ | 5 |
| 33 % CuO, 38 % Cr₂O₃, 9 % BaO | 6 |
| 76 % CuO, 24 % Cr₂O₃ | 7 |
| 72 % CuO, 18 % Cr₂O₃* | 8 |
| 66 % CuO, 25 % Cr₂O₃** | 9 |

| | |
|---|---|
| * Handelsprodukt E-113 T der Firma Mallinckrodt | |
| ** Handelsprodukt T-4421 der Firma Süd-Chemie | |

**Ausführung der Reduktion erfolgte nach folgender an sich üblicher Verfahrensweise:**

Das Katalysatorbett, das sich bereits in dem vorgesehenen Reaktor befindet, wird im Stickstoffstrom auf 150°C aufgeheizt. Bei dieser Temperatur wird langsam Wasserstoff bis zu einer Eingangskonzentration von bis zu 8 Vol% eingespeist. Der Temperaturanstieg der Schüttung sollte nicht größer als 25°C sein (Reduktionsschritt).

Nach Abklingen der Reaktionswärme wird die Wasserstoffkonzentration auf 80 bis 100% gesteigert und die Temperatur auf bis zu 280°C angehoben. Die Temperatur wird 12 h unter H₂-Durchfluß eingehalten (Nachreduktion).

### Verfahrensdurchführungen:

### Ausführungsform A:

Die Katalysatorpellets werden zerkleinert und eine Fraktion von 0,8 bis 1,2 mm ausgewählt. Diese werden in ein mit Silikonöl beheizbares Quarzglasrohr mit 1 cm Innendurchmesser und 30 cm Länge gegeben. Nach Durchführung des Reduktionsschrittes erfolgt die Versuchsausführung. Wasserstoff wird über einen Massendurchflußregler dosiert, der Maleinsäureanhydridpartialdruck über einen sogenannten Sättiger eingestellt. Dies erfolgt dadurch, daß Wasserstoff und ggf. Stickstoff durch den Sättiger, in dem sich flüssiges Maleinsäureanhydrid befindet, geleitet werden und sich aufgrund der genau fixierten Temperatur ein bekannter Maleinsäureanhydrid-Partialdruck eingestellt wird. Das Gemisch wird über beheizte Leitungen dem Reaktor zugeführt. Zur Festlegung der Reaktionstemperatur siehe Ausführungsform C.

### Ausführungsform B:

Diese Ausführungsform ist völlig analog zur Ausführungsform A. Der Unterschied liegt lediglich darin, daß der Reaktor (d.h. Quarzglasrohr) einen Innendurchmesser von 3 cm aufweist und die Pellets in Originalgröße, d.h. unzerkleinert eingesetzt werden (Durchmesser ca. 3 bis 3,2 mm). Zur Festlegung der Reaktionstemperatur siehe Ausführungsform C.

### Ausführungsform C:

Es finden unzerkleinerte Pellets (siehe B) Verwendung. Der Reaktor wurde aus Edelstahl gefertigt; die Abmessungen sind 3 cm Innendurchmesser und 1,2 m Länge. Die Beheizung erfolgt mittels eines Doppelmantels mit Silikonöl, wobei die Temperatur des Silikonöls der Reaktionstemperatur in den Ausführungsbeispielen gleichgesetzt wird. Die Dosierung von Wasserstoff und Stickstoff erfolgt über Massendurchflußregler, kurz vor dem Reaktor erfolgt die Vermischung mit Recycle-Gas, welches nach Abtrennung der flüssigen Produkte wieder dem Reaktor zugeführt wird. Gleichzeitig wird aber aus dem Recycle-Gas ein bestimmter Anteil entnommen und aus dem System ausgeschleußt, um eine Anreicherung von gasförmigen Nebenprodukten zu verhindern. Maleinsäureanhydrid wird flüssig über beheizte Leitungen direkt dem Reaktor zugeführt; als Verdampfungszone dienen die ersten 20 cm des Reaktors, die mit Quarzglaswolle gefüllt sind. Erst im Anschluß daran befindet sich der Katalysator.

### Versuchsbeispiele mit Ausführungsform A:

### A.1.

Die Katalysatoren wurden reduziert wie oben beschrieben. Jeweils 20 ml Schüttvolumen davon wurden in den Reaktor gefüllt und bei einem Durchfluß von 0,38 mol N₂/h und 2 mol H₂/h auf eine Reaktionstemperatur von 275°C gebracht. Dann wurde das Gasgemisch über den Sättiger geleitet und so ein Molenstrom an Maleinsäureanhydrid von 0,02 mol/h eingestellt. Nach 2 h Reaktionszeit wurde das Produktgemisch analysiert. Die Ergebnisse sind in der folgenden Tabelle 2 wiedergegeben.

**Tabelle 2**

| Kat-Nr. | Ausbeuten (in %) | Ausbeuten (in %) | Umsatz (in %) |
|---|---|---|---|
| | gamma-Butyrolacton | Bernsteinsäureanhydrid | Maleinsäure anhydrid |
| 1 | 22.1 | 77,6 | 100 |
| 2 | 49.8 | 49,6 | 100 |
| 3 | 64.9 | 34,2 | 100 |
| 4 | 98.1 | - | 100 |
| 5 | 25.0 | 62 | 100 |
| 6 | 4.7 | 14,0 | 19,5 |
| 7 | 90.9 | 6,9 | 100 |

Daraus ist die besondere Qualität des Katalysators Nr. 4 zu erkennen, wobei von besonderer Bedeutung ist, daß auch das Zwischenprodukt Bernsteinsäureanhydrid vollständig umgesetzt wird, ansonsten sind häufig technische Probleme mit der Kristallisation des Bernsteinsäureanhydrids zu erwarten, die sich in Verstopfung von Rohrleitungen usw. auswirken.

### A.2.

Die im folgenden beschriebenen zwei Versuche wurden ausschließlich mit Katalystor Nr. 4 durchgeführt, und zwar unter Bedingungen, die den Vorteil einer Verwendung von Inertgas verdeutlichen sollen.

10 ml des Katalysators wurden in den Reaktor gefüllt. Der MSA-Strom betrug 0,01 mol/h. Der Gesamtmolenstrom lag bei 1,20 mol/h. Die unterschiedlichen Bedingungen und Ergebnisse verdeutlicht die untenstehende Tabelle. Der MSA-Umsatz war in beiden Fällen 100 %.

**Tabelle 3**

| N₂ (mol/h) | H₂ (mol/h) | Ausbeuten (%): GBL | BSA | T (°C) |
|---|---|---|---|---|
| 0,00 | 1,19 | 97,0 | - | 275 |
| 0,19 | 1,00 | 97,5 | - | 275 |
| 0,49 | 0,70 | 98,7 | - | 275 |
| 0,69 | 0,50 | 87,0 | 11,7 | 275 |

Das heißt, daß zwar bei 275°C in den drei oberen Fällen kein BSA mehr gebildet wurde, die Selektivität und somit Ausbeute zu GBL jedoch mit höherem Inertanteil höher ist.

Bei zu hohem Inertanteil (siehe 4. Beispiel) wird allerdings nicht mehr alles von dem Zwischenprodukt BSA zu GBL hydriert, d.h. nur ein bestimmter Inertanteil ist optimal.

### Versuchsbeispiele mit Ausführungsform B:

### B.1.

Vergleichsbeispiele Kat. Nr. 3 und Nr. 4
Da die Katalysatoren von der Zusammensetzung her eigentlich sehr ähnlich - bis auf den SiO₂-Anteil - aufgebaut sind, wurden folgende Vergleichsversuche durchgeführt. Stickstoff wurde in keinem Fall zugesetzt. Die Katalysatormenge war jeweils 200 g, wobei die ersten 10 cm der Schüttung mit 100 g Quarzglaskugeln mit 1 mm Durchmesser verdünnt waren. Der MSA-Umsatz betrug immer 100 %.

Die untenstehenden Beispiele verdeutlichen, daß die Hydrieraktivität von Nr. 4 ungleich höher ist als von Nr. 3, d.h. bereits der geringe SiO₂-Anteil zu einer signifikant erniedrigten BSA-Bildung führt, was von erheblicher technischer Bedeutung ist.

**Tabelle 4**

| Kat-Nr. | H₂ (mol/h) | MSA (mol/h) | T (°C) | Ausbeute GBL (%) | Ausbeute BSA (%) | Beispiel Nr. |
|---|---|---|---|---|---|---|
| 3 | 15,8 | 0,158 | 270 | 85,7 | 11,0 | 1B |
| 4 | 15,8 | 0,158 | 270 | 85,3 | 0,1 | 2B |
| 3 | 7,87 | 0,105 | 255 | 91,2 | 5,5 | 3B |
| 4 | 7,87 | 0,105 | 255 | 92,5 | 2,0 | 4B |
| 3 | 7,87 | 0,105 | 260 | 89,5 | 6,8 | 5B |
| 4 | 7,87 | 0,105 | 260 | 90,5 | 0,7 | 6B |
| 3 | 15,75 | 0,21 | 270 | 82,8 | 14,3 | 7B |
| 4 | 15,75 | 0,21 | 270 | 91,6 | 1,3 | 8B |
| 3 | 7,87 | 0,105 | 275 | 85,6 | 0,6 | 9B |

Bei Erhöhung der Reaktionstemperatur für Nr. 3 kann dann zwar auch vollständiger BSA-Umsatz erzielt werden, aber auf Kosten erheblicher Selektivitätseinbußen (s. Beispiel 9B).

Vergleicht man Beispiel 4B mit 9B, so kann man bei ähnlicher BSA-Ausbeute eine erheblich höhere Selektivität und Ausbeute an GBL erzielen.

### Versuchsbeispiele mit Ausführungsform C:

### C.1.

In den folgenden Versuchspaaren werden die speziell bei Druckversuchen extrem günstigen Eigenschaften der Inertgasbeimischung auf die GBL-Ausbeute verdeutlicht. (nur Katalysator Nr. 4):

**Tabelle 5**

| MSA-Druck (bar) | Gesamt-Druck (bar) | T (°C) | Kat.-Menge (g) | N₂-Anteil (%) | Verweilzeit (sec) | GBL-Ausbeute (%) |
|---|---|---|---|---|---|---|
| 0,1188 | 6 | 270 | 360 | 0 | 2,69 | 41 |
| 0,1188 | 6 | 270 | 360 | 62 | 2,69 | 86 |
| 0,1131 | 6 | 270 | 625 | 46 | 3,14 | 92 |
| 0,1188 | 6 | 260 | 360 | 0 | 2,74 | 67 |
| 0,1188 | 6 | 260 | 360 | 30 | 2,74 | 83 |

### C.2. Vergleichende Versuche unter Druck

Die technische Überlegenheit des Katalysators Nr. 4 bei Arbeiten unter Druck soll in den folgenden Vergleichsversuchen mit technisch erhältlichen Katalysatoren (Nr. 8 und Nr. 9) nochmal aufgezeigt werden. Am wichtigsten sind dabei die GBL-Ausbeuten sowie die entsprechenden Selektivitäten und die verbliebene Menge an Zwischenprodukt BSA.

**Tabelle 6**

| MSA-Kat.-Druck (bar) | Gesamt-Druck (bar) | T (°C) | Kat.-Menge (g) | N₂-Anteil (%) | Verweilzeit (sec) | GBL-Ausbeute (%) | BSA beute (%) | Nr. |
|---|---|---|---|---|---|---|---|---|
| 0,116 | 6 | 270 | 630 | 0 | 2,69 | 69 | 3,1 | 8 |
| 0,116 | 6 | 270 | 630 | 0 | 2,69 | 75 | 0,2 | 4 |
| 0,116 | 6 | 270 | 630 | 50 | 2,69 | 81 | 13,0 | 8 |
| 0,116 | 6 | 270 | 630 | 50 | 2,69 | 89 | 1,5 | 4 |

Die Versuchsergebnisse zeigen, daß der erfindungsgemäße Katalysator in unerwarteter Weise sich von den übrigen Katalysatoren unterscheidet und zwar sowohl beim Arbeiten mit oder ohne Verdünnungsmittel, bei verschiedenen Temperaturen, bei Druck oder Normaldruck.

Es versteht sich von selbst, daß man durch Auswahl der Reaktionsbedingungen wie Verhältnis der Ausgangsstoffe, Verweilzeit, Temperatur und dergleichen, die Reaktion zusätzlich steuern kann. Die jeweils günstigsten Bedingungen können durch einige einfache Vorversuche, die zum handwerklichen Können eines Durchschnittsfachmannes gehören, bestimmt werden.

## Patentansprüche

1. Verfahren zur Herstellung von gamma-Butyrolacton durch katalytische Hydrierung von Maleinsäureanhydrid in der Dampfphase in Gegenwart von Katalysatoren auf der Basis von Kupferchromit in reduzierter Form, dadurch gekennzeichnet, daß man zur Durchführung der Reaktion einen Katalysator verwendet, der die drei Komponenten Kupferoxyd, Chromoxyd und Siliciumdioxyd enthält.

2. Verfahren nach Anspruch 1, dadurch gekennzeichnet, daß die 3 Komponenten 68 - 85 Gew.% CuO, 15 - 30 Gew.% Cr₂O₃ und 0,5 - 5 % SiO₂ sind, wobei diese Mengenangaben auf den noch nicht durch Reduktion vorbereiteten Katalysator bezogen sind.

3. Verfahren nach Anspruch 2, dadurch gekennzeichnet, daß das Verhältnis CuO zu Cr₂O₃ zu SiO₂ 78 : 20 : 2 ist.

4. Verfahren nach einem der Ansprüche 1 bis 3, dadurch gekennzeichnet, daß die Hydrierung des Maleinsäureanhydrids in Gegenwart eines inerten Gases als Verdünnungsmittel durchgeführt wird.

5. Verfahren nach Anspruch 4, dadurch gekennzeichnet, daß man als inertes Gas Stickstoff verwendet.

6. Verfahren nach einem der Ansprüche 1 bis 5, dadurch gekennzeichnet, daß man den Katalysator durch Reduktion im Reaktor vorbereitet.

7. Verfahren nach einem der Ansprüche 1 bis 6, dadurch gekennzeichnet, daß man Maleinsäureanhydrid und Wasserstoff in einem molaren Verhältnis von 1 : 50 bis 1 : 150 für die Reaktion verwendet.

## Claims

1. A process for the preparation of gamma-butyrolactone by the catalytic hydrogenation of maleic anhydride in the vapour phase and in the presence of catalysts based on copper chromite in reduced form, characterized in that the reaction is carried out using a catalyst containing the three components copper oxide, chromium oxide and silicon dioxide.

2. A process according to Claim 1, characterized in that the 3 components are 68 - 85 wt.% of CuO, 15 - 30 wt.% of Cr₂O₃ and 0.5 - 5% of SiO₂, these proportions being relative to the catalyst before it has been prepared by reduction.

3. A process according to Claim 2, characterized in that the ratio of CuO to Cr₂O₃ to SiO₂ is 78 : 20 : 2.

4. A process according to one of Claims 1 to 3, characterized in that the hydrogenation of the maleic anhydride is carried out in the presence of an inert gas as diluent.

5. A process according to Claim 4, characterized in that nitrogen is used as the inert gas.

6. A process according to one of Claims 1 to 5, characterized in that the catalyst is prepared by reduction in the reactor.

7. A process according to one of Claims 1 to 6, characterized in that maleic anhydride and hydrogen are used for the reaction in a molar ratio of 1 : 50 to 1 : 150.

## Revendications

1. Procédé de préparation de gamma-butyrolactone par hydrogénation catalytique d'anhydride maléique en phase vapeur en présence de catalyseurs à base de chromite de cuivre sous forme réduite, caractérisé en ce que l'on utilise pour la réalisation de la réaction un catalyseur contenant les trois composants que sont l'oxyde de cuivre, l'oxyde de chrome et le dioxyde de silicium.

2. Procédé conforme à la revendication 1, caractérisé en ce que les trois composants sont de 68 à 85 % en poids de CuO, de 15 à 30 % en poids de Cr₂O₃ et de 0,5 à 5 % en poids de SiO₂, ces quantités étant rapportées au catalyeur non encore préparé par réduction.

3. Procédé conforme à la revendication 2, caractérisé en ce que le rapport CuO : Cr₂O₃ : SiO₂ est égal à 78 : 20 : 2.

4. Procédé conforme à une des revendications 1 à 3, caractérisé en ce que l'on réalise l'hydrogénation de l'anhydride maléique en présence d'un gaz inerte jouant le rôle d'agent de dilution.

5. Procédé conforme à la revendication 4, caractérisé en ce que l'on utilise comme gaz inerte de l'azote.

6. Procédé conforme à une des revendications 1 à 5, caractérisé en ce que l'on prépare le catalyseur par réduction dans le réacteur.

7. Procédé conforme à une des revendications 1 à 6, caractérisé en ce que l'on utilise pour la réaction de l'anhydride maléique et de l'hydrogène en un rapport molaire compris entre 1 : 50 et 1 : 150.
